(19) Europäisches Patentamt
European Patent Office
Office européen des brevets

(11) **EP 3 884 958 A1**

(12) **EUROPEAN PATENT APPLICATION**
published in accordance with Art. 153(4) EPC

(43) Date of publication:
**29.09.2021 Bulletin 2021/39**

(21) Application number: **18928855.8**

(22) Date of filing: **31.07.2018**

(51) Int Cl.:
*A61K 39/00* (2006.01)        *A61P 35/00* (2006.01)
*A61K 41/00* (2020.01)        *C12N 5/09* (2010.01)
*A61K 38/00* (2006.01)

(86) International application number:
**PCT/IB2018/055740**

(87) International publication number:
**WO 2020/026001 (06.02.2020 Gazette 2020/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Pineda Olvera, Benjamín**
  **Ciudad De México, 14269 (MX)**

• **Pérez de la Cruz, Verónica**
  **Ciudad De México, 14269 (MX)**

(72) Inventors:
• **Pineda Olvera, Benjamín**
  **Ciudad De México, 14269 (MX)**
• **Pérez de la Cruz, Verónica**
  **Ciudad De México, 14269 (MX)**

(74) Representative: **Padial Martinez, Ana Belen**
  **Arochi & Lindner**
  **C/ Gurtubay Nº 6-3º Izquierda**
  **28001 Madrid (ES)**

(54) **VACCINATION WITH MICROVESICLES DERIVED FROM TUMOUR CELLS FOR CANCER TREATMENT**

(57)    The present invention relates to microvesicles derived from natural tumor cells and tumor cells produced in vitro under a stress stimulus, such as radiation, which can be used in an effective manner as a therapeutic vaccine for cancer. The invention also relates to a therapeutic vaccine formulation containing the microvesicles, processes for the preparation and medical use thereof as a therapeutic vaccine to stimulate the antitumor immune system and treat cancer.

EP 3 884 958 A1

## Description

## TECHNICAL FIELD OF THE INVENTION

The present invention relates to the field of health, specifically with the area of brain cancer and other neoplasms with the potential for treatment with microvesicles from tumor cells.

## BACKGROUND

Gliomas are the most common primary brain tumors in adults and the second cause of cancer in children after leukemia with an incidence of 22 cases per 100,000 inhabitants (1), causing more than 15,000 deaths each year in the United States alone (2). The World Health Organization (WHO) classifies them as astrocytomas, oligoastrocytomas, and oligodendrogliomas, according to their histological similarity to the glial cells. Based on their biological behavior and degree of malignancy, they are divided into 4 grades (I to IV), with glioblastoma (GBM) being the most severe (3) and the most common among all malignant tumors of the brain and the Central Nervous System (4). In the National Institute of Neurology and Neurosurgery of Mexico (INNN, per its acronym in Spanish), GBM represents 9% of all brain tumors and 45.7% of primary gliomas, with a mean of 51 years and a greater frequency in men than in women (1.8:1) (5,6).

The current standard treatment for GBM consists of extensive surgical resection, followed by radiotherapy and chemotherapy, the latter based on the use of Temozolamide; however, the mean survival is 14.6 months and only 5.1% of patients survive more than 5 years, a perspective that has not changed in the last two decades (4, 7). Currently, new therapeutic strategies have focused on better understanding the biology of brain tumors, wherein an area vaguely studied in neuro-oncology is extracellular vesicular trafficking in the form of extracellular vesicles (EV) (8).

During the last decade, research on extracellular vesicles has increased significantly and its application in anti-tumor vaccination has been considered (9). These vesicles are lipid bilayer membrane particles that are released from most cells, including tumor cells, constitutively or in response to stress, and can be isolated from various body fluids such as: blood, urine, saliva, breast milk, amniotic fluid, ascites, semen, and cerebrospinal fluid (10).

The release of EV was originally reported by Chargaff and West in 1946 as platelet-derived pro-coagulant particles in normal plasma (11) and later referred to as "platelet dust" by Wolf in 1967, who noted coagulant material in the form of small particles, sedimentable by high-speed centrifugation and surrounding activated platelets (12). In 1978, EVs were documented for the first time in a cancer patient when they were identified by electron microscopy in spleen and lymph node cultures from a person with Hodgkin's disease (13).

Microvesicles (MV) (also referred to as microparticles or ectosomes) represent a specific subtype of EV. They are heterogeneous particles in size, ranging from 200 nm to more than 1 $\mu$m and are released into the extracellular space by fission towards the outside of the plasma membrane (14, 15). The exact mechanism by which MVs are generated has not been fully understood, but a loss of membrane lipid asymmetry is known to occur (16). At the site where the microvesicles are released, the phosphatidylserine amiophospholipid, normally found on the cytoplasmic side of the membrane, is relocated to the outer layer while the topology of the membrane proteins remains intact (17).

The importance of MVs lies in their ability to transfer their content to other cells locally or systemically. Once produced by the donor cell, MVs can: 1) bind via receptors to the surface of a target cell; 2) fuse with the target cell membrane and discharge its contents into the cytosol; or 3) be taken up by the cell through endocytosis, which after being endocytized, can remain isolated within the endosomes and, ultimately, fuse with the lysosomes. Alternatively, they can fuse their membrane with that of the endosome and subsequently discharge their contents to the cytosol (horizontal transfer) or be expelled intact to the outside (transcytosis) (14).

The composition of the microvesicles largely depends on the type of cell from which they originated, although the composition of the membrane of the microvesicles is different from that of the parent cell, with significant remodeling (15). The molecules present in MVs involved in cancer contain a plethora of bio-active molecules that include antigens involved in immunomodulation, transmembrane receptors and ligands, oncoproteins and tumor suppressor proteins, lipids, messenger RNA, microRNA, and genomic and mitochondrial DNA (18). These MVs can fuse and interact with multiple cell types modifying and establishing pre-metastatic niches, cell invasion, angiogenesis (19) and innate immune modulation (20).

On the other hand, it has been noted that the irradiation of gliomas alters the abundance and composition of MVs promoting a migratory phenotype (19). Recently, Baulch et al. (21) demonstrated that radiation triggers a pro-oxidant phenotype that favors marked changes in the expression of genes involved in regulating cellular reprogramming and MVs-mediated paracrine interactions that promote metalloproteinase 2 (MMP-2)-mediated survival and invasiveness. Furthermore, ionizing radiation has been shown to induce the release of MVs and contribute to the formation of damage-associated molecular patterns, called damage-associated molecular patterns (DAMPs) (22). DAMPs can be released both passively by necrotic cells and be secreted or exposed by living cells when they are subjected to stress that threatens their survival, which leads to stimulating the cells of the innate immune system that is responsible for detecting pathogen-associated molecular patterns (PAMPs) or DAMPs through pattern recognition receptors (PRRs) (23).

At present, clinical studies have been carried out in cancer patients where EV derived from neoplastic cells have been used. In a phase I study, carried out in 15 patients with stage III/IV metastatic melanoma, the viability of the production of EV from autologous dendritic cells derived from monocytes loaded with the MAGE3 peptide, as well as the safety of its subcutaneous delivery; without significant toxicity (> grade II) (24). In another Phase I study, peptide-loaded monocyte-derived dendritic cell EVs subcutaneously and intradermally delivered were used in 13 patients with advanced non-small cell lung cancer; therapy was well tolerated (toxicity grade I-II) and some patients experienced long-term disease stability and activation of immune effectors (25). Dai et al. conducted a Phase I clinical study with 40 patients with stage III and IV colon cancer who were treated with ascites fluid-derived autologous EVs subcutaneous route, alone or accompanied by GM-CSF, where its safety and induction of specific cytotoxic T responses against the tumor was shown.

On the other hand, Graner et al. (8) demonstrated in a murine glioblastoma model that prophylactic vaccination with exosomes (containing MVs) induced activation of the immune system by stimulating humoral and cellular immunity by T cells, thus avoiding tumor implantation and favoring a long term memory response; however, when using these exosomes in therapeutic vaccination, they were not able to modify the survival of mice implanted intracerebrally, despite inducing a cellular and humoral immune response.

The conventional treatment in cancer consists of surgery, radiation and chemotherapy; however, the very unfavorable prognosis in many of these neoplasms reveals the need to develop new therapies, such as immunotherapeutic strategies. Microvesicles are produced by almost all cells, including tumor cells, and have a preponderant role in the transfer of intercellular information, having the capacity to suppress the immune system, increase tumor progression, promote invasiveness, metastasis and confer multidrug resistance to neighboring neoplastic cells. Given the plethora of bioactive antigens contained therein, it allows increasing the possibility that they deliver a greater tumor antigenic repertoire, which makes them candidates for use as therapeutic vaccines against cancer.

## BRIEF DESCRIPTION OF THE INVENTION

As a result of extensive research and development work, the inventors of the present patent application have unexpectedly found that microvesicles produced by in vitro irradiated neoplastic cells can be used effectively as a therapeutic vaccine for cancer.

In a first aspect, the present invention relates to said microvesicles that come from neoplastic cells irradiated in vitro.

In a second aspect, the present invention relates to microvesicles produced by in vitro irradiated tumor cells for use, alone or in combination with one or more antineoplastic treatments, in the treatment or prophylaxis of malignant neoplasms, in the modulation of the antitumor immune response, and as prognostic or diagnostic markers in any type of neoplasm.

In a third aspect, the invention relates to a therapeutic vaccine for cancer comprising the microvesicles produced by in vitro irradiated tumor cells and pharmaceutically acceptable additives.

Finally, in a fourth aspect, the present invention relates to a process for preparing the microvesicles of the present invention or the therapeutic vaccine of the present invention, wherein said process comprises the step of irradiating neoplastic cells.

The present invention is one of many attempts, seeking to create a simple and low-cost immunotherapeutic alternative that improves the expectation of current treatment as an adjuvant or as a single therapy, allowing a better life expectancy and quality of life for patients with various neoplasms.

## DESCRIPTION OF THE DRAWINGS

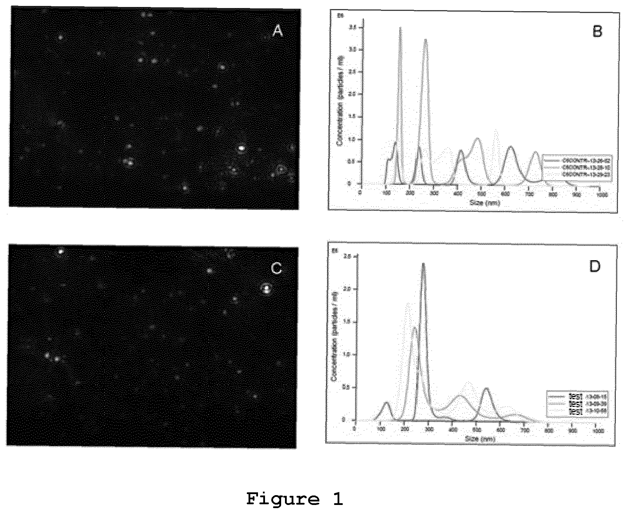

Figure 1 shows the results of the determination of the size of the microvesicles. Representative images of the size distribution of C6 (non-irradiated) (A) and irradiated C6 (C) microvesicles, obtained from videos of NANOSIGHT nanoparticle scan analysis (left panel), and analysis of the size distribution of the C6 microvesicles (B) and irradiated C6 (D) (in triplicate) using the software NANOSIGHT NTA 3.2 Dev Build 3.2.16 (right panel).

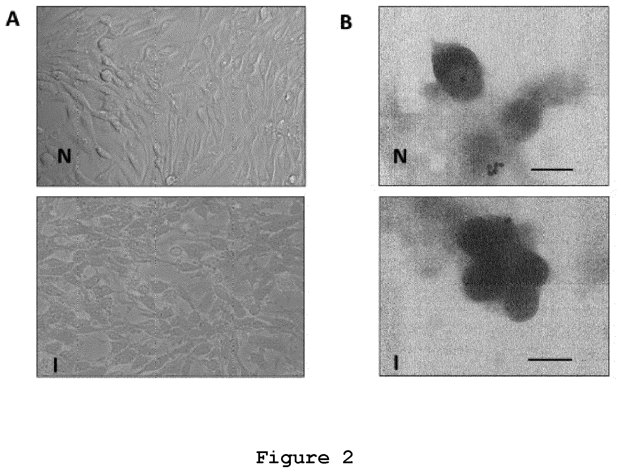

Figure 2 shows the results of the microscopy of irradiated and non-irradiated C6 glioma cells, as well as microvesicles thereof. On the left side (A) of the figure, brightfield microscopy images of C6 glioma cells (N) and C6 glioma cells (I) irradiated with 50Gy radiation dose (20X) can be seen. On the right side (B), electron microscopy images of transmission of non-irradiated C6 cells microvesicles (N) and irradiated C6 cells microvesicles (I) labeled with annexin V-gold are seen. The bar in the image represents 200 nm.

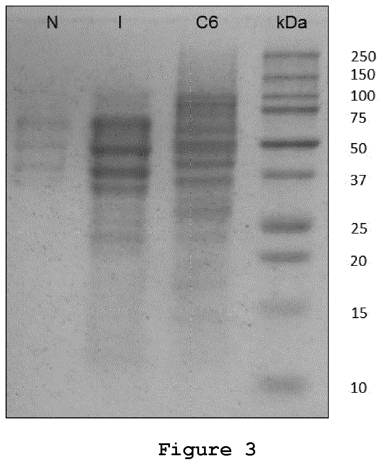

Figure 3 shows the results of 15% polyacrylamide gel denaturing electrophoresis of proteins obtained from C6 cells (C6), non-irradiated C6 cell microvesicles (N) and irradiated C6 cell microvesicles (I). KDa represents the molecular weight marker.

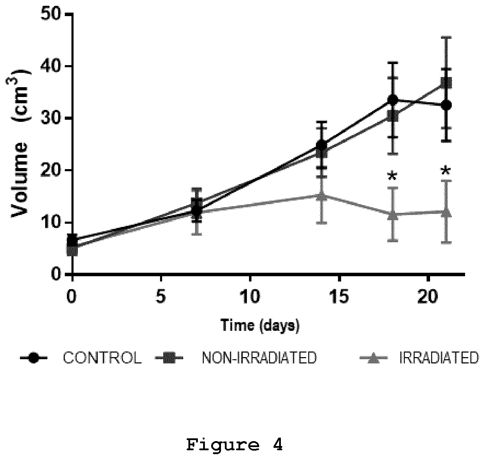

Figure 4 shows the results of the tumor growth kinetics. One million viable C6 cells were implanted subcutaneously into Wistar rats. Once the tumors had developed (≥2 cm in diameter), the rats were administered subcutaneously with C6 MV, irradiated C6 MV (50 Gy) or PBS on days 0 and 7. Then, the tumor volume was determined at days 0,7, 14, 18 and 21. Results are expressed as mean ±SEM. * p=0.031 irradiated vs control.

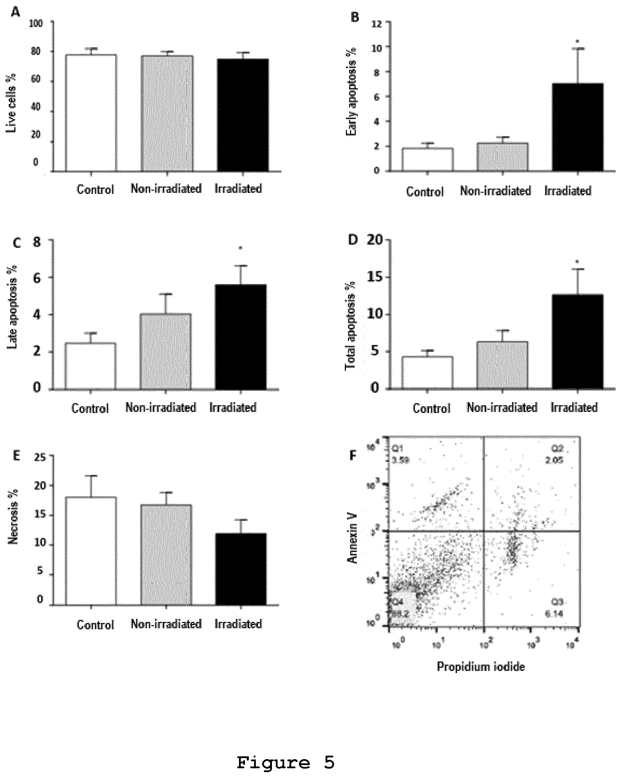

Figure 5 shows the results of a tumor apoptosis and necrosis evaluation. For this purpose, the following were evaluated by flow cytometry: (A) live cells (negative for annexin V and negative for propidium iodide), (B) cells in early apoptosis (positive only for annexin V), (C) late apoptosis (positive for annexin V and propidium iodide), (D) total cells in apoptosis and (E) necrosis (positive for propidium iodide), in tumors of rats with subcutaneous glioblastoma, 21 after treatment with non-irradiated C6 MV, irradiated C6 MV (50 Gy) or PBS (control group). A representative dot plot of the populations is presented (F). The results are expressed as the mean ±SEM, early apoptosis *p=0.027, late apoptosis *p=0.022, total apoptosis *p=0.038, for irradiated vs. control.

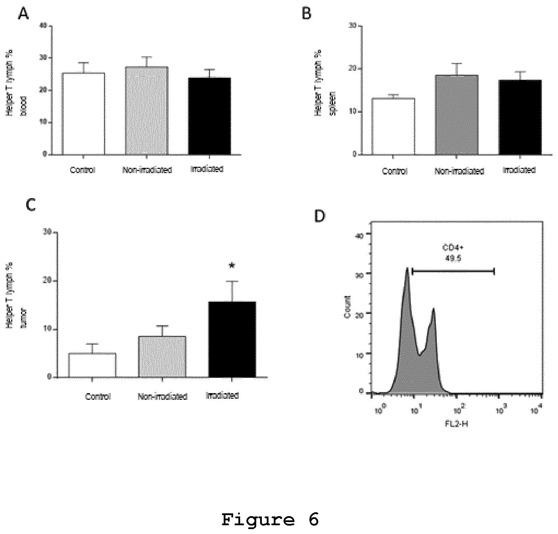

Figure 6 shows the results of a comparative study of helper T lymphocytes. Flow cytometric analysis of CD4+ cells was performed in (A) blood, (B) spleen and (C) tumor of rats with subcutaneous glioblastoma, 21 after treatment with non-irradiated C6 MV, irradiated C6 MV (50 Gy) or PBS (control group). A representative histogram of CD4+ T lymphocytes in blood (D) is presented. The results are expressed as the mean ±SEM. *p=0.036 irradiated vs control.

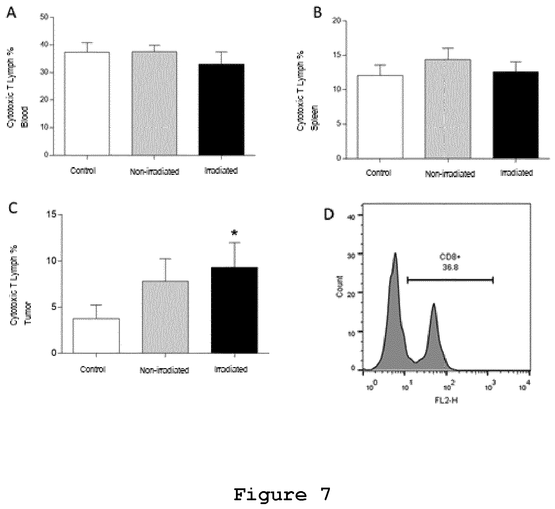

Figure 7 shows a comparative study of cytotoxic T lymphocytes. Flow cytometric analysis of CD8+ cells was performed in (A) blood, (B) spleen and (C) tumor of rats with subcutaneous glioblastoma, 21 after treatment with non-irradiated C6 MV, irradiated C6 MV (50 Gy) or PBS (control group). A representative histogram of CD8+ T cells in blood is presented (D). The results are expressed as the mean ±SEM. *p=0.04 irradiated vs control.

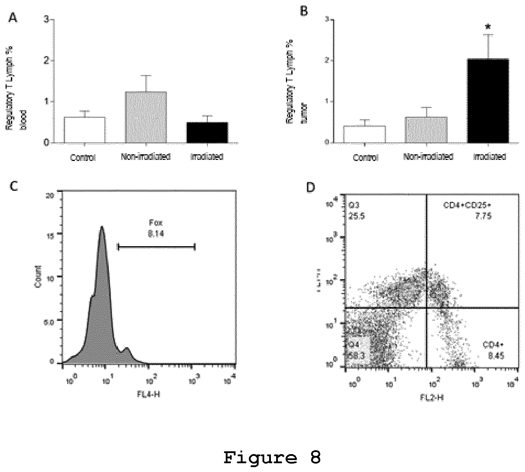

Figure 8 shows a comparative study of regulatory T lymphocytes. Flow cytometric analysis of CD4+/CD25+/FoxP3+ cells was performed in (A) blood and (B) tumor of rats with subcutaneous glioblastoma, 21 after treatment with non-irradiated C6 MV, irradiated C6 MV (50 Gy) or PBS (control group). A representative dot plot is presented for the selection of the CD4+/CD25+ lymphocyte population in blood (D), from which the amount of regulatory T lymphocytes (FoxP3+) (E) was determined. The results are expressed as the mean ±SEM. *p=0.037 irradiated vs control.

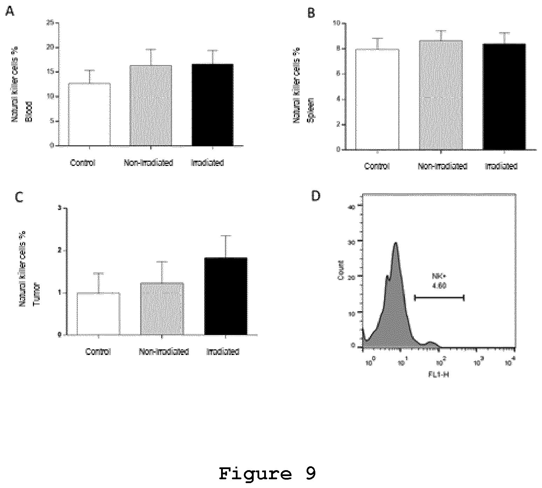

Figure 9 shows a comparative study of natural killer (NK) cells. Flow cytometry analysis of NKR-P1+ cells in (A) blood, (B) spleen and (C) tumor of rats with subcutaneous glioblastoma was performed, 21 after treatment with non-irradiated C6 MV, irradiated C6 MV (50 Gy) or PBS (control group). A representative histogram of blood NK cells is presented (D). The results are expressed as the mean ± SEM, no significant differences were noted.

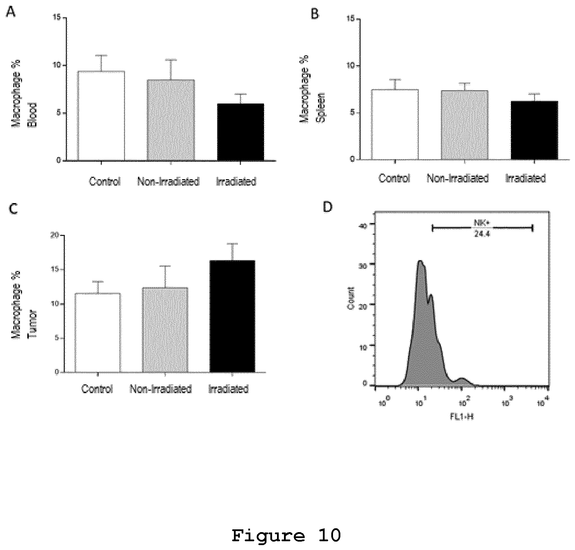

Figure 10 shows a comparative study of macrophages. Flow cytometric analysis of CD68+ cells was performed in (A) blood, (B) spleen and (C) tumor of rats with subcutaneous glioblastoma, 21 after treatment with non-irradiated C6 MV, irradiated C6 MV (50 Gy) or PBS (control group). The results are expressed as the mean ±SEM, no significant differences were noted.

## DETAILED DESCRIPTION OF THE INVENTION

In a first aspect, the invention relates to microvesicles that come from in vitro irradiated neoplastic cells, which are formed by a lipid bilayer and membrane proteins can be found on their surface, such as growth factor receptors, integrin receptors and MHC class I molecules. Inside same, soluble proteins can be found such as proteases and cytokines, as well as various nucleic acids. In addition, they may contain a plethora of bioactive molecules present in cancer cell-derived microvesicles, including antigens involved in immunomodulation, transmembrane receptors and ligands, oncoproteins and tumor suppressor proteins, lipids, mRNA, microRNA, and genomic and mitochondrial DNA.

The microvesicles according to the present invention have an mean size between 200 and 400 nm and, preferably, the mean size is of about 340 nm, taking into account the data obtained from a nanoparticle scan analysis (Nanosight).

The radiation dose that can be applied to obtain the microvesicles of the present invention is in the range between 45 and 55 Gy, and preferably the radiation dose is 50 Gy.

The microvesicles of the present invention can be further characterized because they comprise HSP70 and/or HSP90 heat shock proteins, since the applied radiation induces the production of heat shock proteins that will be contained only in the microvesicles derived from radiated cells and not in non-radiated microvesicles.

Several publications (27, 28, 29, 30 and 31) have confirmed that after the radiation of tumor cells (particularly from glioblastoma, including C6 cells) the heat shock protein HSP70 is overexpressed, from doses as low as 2Gy and even passing from the cytoplasm to the membrane.

The microvesicles of the present invention can contain HSP70 (HSP70 +) and/or HSP90 (HSP90 +), and further have phosphatidylserine in the outer membrane, which is why they are positive to annexin V (annexin V +), but negative to NFATC4 (NFATC4-).

Therefore, the microvesicles according to the first aspect of the invention can alternatively be characterized because the outer membrane of the microvesicles contains phosphatidylserine, because they are positive to annexin V and/or because they do not contain Nuclear Factor of Activated T Cell 4 (NFATC4).

In a second aspect, the present invention relates to the use of microvesicles derived from in vitro irradiated neoplastic cells according to the first aspect of the present invention, alone or in combination with one or more antineoplastic treatments, for the preparation of a therapeutic vaccine for the treatment or prophylaxis of malignant neoplasms, or for the modulation of the anti-tumor immune response.

Microvesicles according to the second aspect of the invention can also be characterized by a mean size of between 200 and 400 nm, and more preferably the mean size is of about 340 nm. The radiation dose applied to in vitro irradiated neoplastic cells can be between 45 and 55 Gy, and more preferably of 50 Gy.

According to this second embodiment, the microvesicles can be further characterized because they do not contain activated Nuclear Factor of Activated T Cell 4 (NFATC4), because they comprise HSP70 and/or HSP90 heat shock proteins, because the outer membrane of the microvesicles contains phosphatidylserine and/or because they are positive to annexin V.

In yet another embodiment of the present aspect, the invention relates to the microvesicles of the present invention, for use alone or in combination with one or more antineoplastic treatments, in the treatment or prophylaxis of malignant neoplasms, or in the modulation of the antitumor immune response.

The one or more above cited antineoplastic treatments can be selected from the group including chemotherapy, radiotherapy, immunotherapy or combinations thereof.

In a further embodiment, the invention also relates to the microvesicles of the invention for use as prognostic or diagnostic markers in any kind of neoplasm.

The microvesicles of the invention can be obtained from tumor cells from cell lines or autologous primary cultures (of the same type of the cancer to be treated, for example, human glioblastoma cells in culture for treatment of patients diagnosed with glioblastoma) previously irradiated can be delivered to patients with solid or liquid tumors by subcutaneous or intradermal route, preferably in a region close to the tumor site.

They can also be delivered intranodally, preferably in lymphoid nodules that drain the tumor in order to increase the antitumor immune response of cancer patients.

The microvesicles according to the present invention can be delivered alone or in combination with radiotherapy, chemotherapy or with other modifiers of the immune response, for example adjuvants, microvesicles-loaded dendritic cells, antitumor monoclonal antibodies or immunotoxins, modifiers of the antitumor immune response; as well as with antitumor immune cells such as helper, cytotoxic T lymphocytes, NK cells, dendritic cells, expanded in vitro with or without any modification, among others; likewise, it can be used in combination with other biological therapies such as oncolytic viruses or transgenes.

In the third aspect, the invention relates to a therapeutic cancer vaccine comprising the microvesicles produced by irradiated tumor cells according to the first aspect of the invention, along with pharmaceutically acceptable additives.

For the purposes of this patent application, the term "additives" refers to any substance that is included in the formulation of the drug and that acts as a vehicle, preservative or modifier of any of the characteristics thereof to enhance its efficacy, safety, stability, appearance. or acceptability. This term includes, interchangeably, the pharmaceutically acceptable excipients, vehicles or carriers that may be contained in the therapeutic vaccine of the present invention.

Therefore, the therapeutic vaccine of the present invention can contain gelatin, albumin, sacarose, lactose, sodium glutamate and glycine. They can also contain diluents, for example, water or saline solution; preservatives or preservers such as thimerosal, phenoxyethanol, and formaldehyde; stabilizers, for example monosodium glutamate (MSG), 2-phenoxyethanol, partially hydrolyzed gelatin and collagen generally sourced from bovine or porcine; antibiotics and/or adjuvants such as aluminum salts: aluminum hydroxide, potassium aluminum sulfate and aluminum phosphate.

In the fourth aspect, the invention relates to a process for preparing the microvesicles according to the first aspect of the invention that comprises the step of irradiating neoplastic cells in vitro, preferably at a radiation dose of between 45 and 55 Gy, and more preferably the radiation dose is 50 Gy.

The process of the present invention also includes the preparation of the therapeutic vaccine according to the second aspect of the invention.

The microvesicles of the present invention can be obtained from neoplastic cells derived from cell lines in culture, primary cultures, serum, urine or any other fluid from which they can be isolated. They can be produced and released naturally by neoplastic cells into the extracellular space or by stressors according to the process of the fourth aspect of the present invention, or along with chemical agents or other physical factors, and subsequently isolated by centrifugation at 14,000 x g.

Also, the microvesicles of the present invention can be isolated by using substances with recognition of phosphatidylserine such as Annexin V bound to magnetic beads and subsequent capture by means of a magnetic field or by flow cytometry or any other microvesicle isolation system, according to the standard methods known in the prior art.

The following example illustrates the best method known to the applicant for carrying out the invention. Furthermore, it exemplifies the industrial application of the invention and is provided for the purposes of illustrating the invention and not to limit the scope of the claims.

## EXAMPLE

The inventors of the present patent application decided to experimentally test the effect of microvesicles from in vitro non-radiated rat glioblastoma cells and glioblastoma cells irradiated with 50 Gy in a subcutaneous model of rat glioblastoma for the induction of an antitumor immune response.

### Cell cultures

Rat C6 glioma cells were obtained from the American Culture and Tissue Collection (Rockville, MD, USA). The cells were cultured in sterility at 37°C in a humid atmosphere controlled with 5% CO2 in Dulbecco's modified Eagle medium (DMEM) (GIBCO BRL) supplemented with 10% fetal bovine serum (GIBCO, BRL.), 4 mM glutamine, 100 units/ml penicillin, and 100 mg/ml streptomycin. Before being used, the supplemented medium was filtered with a 0.22 $\mu$m GSWP membrane (Millipore) to eliminate possible contaminating MVs.

The general cell culture procedure can be used for any tumor line from which microvesicles are to be obtained or it can be modified depending on the supplier's specifications or the type of primary culture to be used.

### Isolation of the microvesicles

The MVs were obtained from cultures of C6 cells and C6 cells irradiated with a dose of 50 Grays (Gy) using a Novalis linear accelerator (Varian and Brainlab) that generates a 6 mega volt X-ray beam, other equipment can be used that allow us to achieve a radiation dose of 50 Gy. After 72 h of radiation, the culture medium was collected and centrifuged twice at a 500 xg speed for 10 minutes at 4°C to eliminate viable cells and cellular debris.

Subsequently, the supernatant was centrifuged at 14,000 xg for 20 minutes at 4°C to settle the microvesicles, which were immediately washed, resuspended in PBS and frozen at -70°C until use, in the same way these MVs can be preserved by fixing them in 1% paraformaldehyde in PBS for 20 minutes and subsequently washing them with PBS and centrifuging them again at 14,000 xg for 20 minutes at 4°C to settle them.

### Microvesicle quantification

For the quantification thereof, the microvesicles were stained with Annexin V-FITC (Annexin-VFLUOS Staining Kit, Roche), which has a high affinity for phosphatidylserine, and analyzed by flow cytometry (FACSCalibur, BD Bioscience) considering the acquisition rate, (acquired volume/time), acquiring 30 seconds per sample and the number of events acquired during that time and the percentage of annexin V-FITC positive events. For data analysis, the Cell QuestPro (BD Bioscience) and Flow Jo version 10 programs were used.

### Characterization of the microvesicles.

Transmission electron microscopy, nanoparticle screening analysis and electrophoresis on polyacrylamide gels were used for the characterization to determine the protein content as follows.

### Transmission electron microscopy

Once collected, the microvesicles were stained with the Annexin V-gold conjugate (15 nm, Biorbyt) for 30 minutes, they were centrifuged at 14,000 xg for 20 minutes at 4°C and the pellet was resuspended in PBS. Subsequently, 10 $\mu$l of the suspension were placed on carbon-coated nickel grids and formed for 20 minutes. The grid was dried on filter paper, stained with 2% uranyl acetate for 5 minutes. The grid was removed from the uranyl acetate and washed with distilled water for one minute. The microvesicles were observed with a JEOL 1010 transmission microscope.

### Nanoparticle screening analysis

The size of the microvesicles was examined by nanoparticle screening analysis with a NanoSight (NanoSight Ltd Amesbury, UK) equipped with a blue laser (488 nm) and a sCMOS camera. The preparations were measured in triplicate (temperature 22.0°C; viscosity 0.95 cP) for 10 s. The software used for data capture and analysis was NTA 3.2 Dev Build 3.2.16. The following results were obtained :

| Microvesicles mean size determined by NANOSIGHT | |
|---|---|
| Microvesicles source | Mean Size (nm) $\pm$ SD |
| C6 | 395.0 $\pm$ 202.6 |
| Irradiated C6 | 339.9 $\pm$ 139.0 |

### Protein content analysis

Protein extraction was performed from C6 cells, C6 microvesicles and irradiated C6 microvesicles using the ProteoJET™ Cytoplasmic and Nuclear Protein Extraction Kit (Fermentas), and were subsequently quantified by the Lowry method. The proteins (30 mg) were electrophoresed under denaturing conditions on a 15% polyacrylamide gel. Precision Plus Protein Standards (Bio-Rad) was used as molecular weight marker. Once the electrophoresis was finished, the gel was stained with Coomassie blue.

To verify the effectiveness, the following experimental design was carried out:

Thirty rats, which were inoculated with C6 cells and whose tumor had a diameter of not less than 2 cm, were separated into three groups (n = 10): the first group was administered with PBS (control group), the second group was treated with C6 cell microvesicles and the third group with irradiated C6 cell microvesicles (1 $\times$ 10 microvesicles). Treatments and PBS were emulsified with Freund's complete adjuvant at a 1:1 ratio and delivered subcutaneously in the thigh that was contralateral to the tumor in rats. A second delivery (booster) was made 7 days after the first delivery. The initial tumor volume was recorded and evaluated at days 7, 14, 18 and 21 by measuring the three diameters of the tumor with a calibrated vernier. The animals were sacrificed 21 days after the treatment by exsanguination (previous anesthesia with Ketamine/Xylazine) and the tumor, blood and spleen were collected for analysis.

Determination of tumor volume

Tumor volume ($cm^3$) was calculated for each rat and time using the following formula described by Tomayko & Reynolds, 1989 (26):

*Tumor volume = $\pi$/6 length x width x height.*

Evaluation of macrophage, NK cell and T lymphocyte populations

The percentages of helper T (CD4 +), cytotoxic T (CD8 +) and regulatory T (CD4+/CD25+/FoxP3+) lymphocytes, as well as natural killer cells (NKR-P1+) and macrophages (CD68+) were determined in blood, spleen and tumor samples by flow cytometry using the anti-CD4-PE rat, anti-CD8-PE rat, anti-NKR-PI-FITC rat, anti-CD25-FITC rat, anti-Foxp3-APC rat, and anti-CD68 rat monoclonal antibodies, the latter together with a secondary antibody coupled to APC.

Briefly, 30 $\mu$L of blood or spleen or tumor homogenate were incubated with 5 $\mu$L of the corresponding monoclonal antibody (1:100 dilution) for 30 minutes. Subsequently, 200 $\mu$L of red blood cell lysis solution (BD Bioscience) were added, the samples were incubated for 10 minutes and washed with PBS. For regulatory T lymphocytes, 200 $\mu$L of permeabilization solution (BD Bioscience) were also added, incubated for 10 minutes, washed and incubated with anti-FoxP3-APC for 30 min. All cells, after being washed with PBS, were fixed with 1% paraformaldehyde in PBS. The cells were analyzed with a FACSCalibur kit (BD Biosciences) using the CellQuest Pro (BD Bioscience) and Flow Jo version 10 programs.

Determination of apoptosis and necrosis

A part of the tumor homogenate was taken to evaluate apoptosis and necrosis. Cells were washed with PBS and stained with Annexin V and propidium iodide (Annexin-VFLUOS Staining Kit, Roche) in 100 $\mu$L of binding buffer for 15 minutes in the dark at room temperature. Subsequently, additional 200 $\mu$L of binding buffer were added and analyzed by flow cytometry (FACSCalibur, BD Biosciences) with Cell QuestPro (BD Biosciences) and Flow Jo version 10.

Statistical analysis

The Shapiro-Wilk normality test was performed on the data. Subsequently, each treatment was compared with the control using t-student or U-Mann, as appropriate. A value of $p \leq 0.05$ was considered significant. For this analysis, the SPSS Statistic 23.0 program (IBM SPSS Statistics for Windows, Version 23.0. Armonk, NY: IBM Corp.) was used.

References

1. Ostrom QT, Gittleman H, Fulop J, Liu M, Blanda R, Kromer C, et al. CBTRUS Statistical Report: Primary Brain and Central Nervous System Tumors Diagnosed in the United States in 2008-2012. Neuro-oncology. 2015;17 Suppl 4:iv1-iv62.
2. Deorah S, Lynch CF, Sibenaller ZA, Ryken TC. Trends in brain cancer incidence and survival in the United States: Surveillance, Epidemiology, and End Results Program, 1973 to 2001. Neurosurgical focus. 2006;20(4):E1.
3. Louis DN, Perry A, Reifenberger G, von Deimling A, Figarella-Branger D, Cavenee WK, et al. The 2016 World Health Organization Classification of Tumors of the Central Nervous System: a summary. Acta Neuropathol. 2016;131(6):803-20.
4. Ostrom QT, Gittleman H, Fulop J, Liu M, Blanda R, Kromer C, et al. CBTRUS Statistical Report: Primary Brain and Central Nervous System Tumors Diagnosed in the United States in 2008-2012. Neuro-Oncology. 2015;17(suppl 4):iv1-iv62.
5. Lopez-Gonzalez MA, Sotelo J. Brain tumors in Mexico: characteristics and prognosis of glioblastoma. Surgical neurology. 2000;53(2):157-62.
6. Velásquez-Pérez L, Jiménez-Marcial ME. Clinical-histopathologic concordance of tumors of the nervous system at the Manuel Velasco Suarez National Institute of Neurology and Neurosurgery in Mexico City. Arch Pathol Lab Med. 2003;127(2):187-92.
7. Stupp R, Mason WP, van den Bent MJ, Weller M, Fisher B, Taphoorn MJB, et al. Radiotherapy plus Concomitant and Adjuvant Temozolomide for Glioblastoma. New England Journal of Medicine. 2005;352(10):987-96.
8. Graner MW, Alzate O, Dechkovskaia AM, Keene JD, Sampson JH, Mitchell DA, et al. Proteomic and immunologic analyses of brain tumor exosomes. FASEB journal : official publication of the Federation of American Societies for Experimental Biology. 2009;23(5):1541-57.
9. Vader P, Breakefield XO, Wood MJ. Extracellular vesicles: emerging targets for cancer therapy. Trends Mol Med. 2014;20(7):385-93.
10. Lee Y, El Andaloussi S, Wood MJ. Exosomes and microvesicles: extracellular vesicles for genetic information transfer and gene therapy. Hum Mol Genet. 2012;21(R1):R125-34.

11. CHARGAFF E, WEST R. The biological significance of the thromboplastic protein of blood. J Biol Chem. 1946;166(1):189-97. PubMed PMID: 20273687.

12. Wolf P. The nature and significance of platelet products in human plasma. Br J Haematol. 1967;13(3):269-88.

13. Friend C, Marovitz W, Henie G, Henie W, Tsuei D, Hirschhorn K, et al. Observations on cell lines derived from a patient with Hodgkin's disease. Cancer Res. 1978;38(8):2581-91.

14. Cocucci E, Racchetti G, Meldolesi J. Shedding microvesicles: artefacts no more. Trends Cell Biol. 2009;19(2):43-51.

15. Muralidharan-Chari V, Clancy J, Plou C, Romao M, Chavrier P, Raposo G, et al. ARF6-regulated shedding of tumor cell-derived plasma membrane microvesicles. Curr Biol. 2009;19(22):1875-85.

16. Zwaal RF, Schroit AJ. Pathophysiologic implications of membrane phospholipid asymmetry in blood cells. Blood. 1997;89(4):1121-32.

17. Lima LG, Chammas R, Monteiro RQ, Moreira ME, Barcinski MA. Tumor-derived microvesicles modulate the establishment of metastatic melanoma in a phosphatidylserine-dependent manner. Cancer Lett. 2009;283(2):168-75.

18. Lee TH, D'Asti E, Magnus N, Al-Nedawi K, Meehan B, Rak J. Microvesicles as mediators of intercellular communication in cancer--the emerging science of cellular 'debris'. Semin Immunopathol. 2011;33(5):455-67.

19. Arscott WT, Tandle AT, Zhao S, Shabason JE, Gordon IK, Schlaff CD, et al. Ionizing radiation and glioblastoma exosomes: implications in tumor biology and cell migration. Translational oncology. 2013;6(6):638-48.

20. Ratajczak J, Miekus K, Kucia M, Zhang J, Reca R, Dvorak P, et al. Embryonic stem cell-derived microvesicles reprogram hematopoietic progenitors: evidence for horizontal transfer of mRNA and protein delivery. Leukemia. 2006;20(5):847-56.

21. Baulch JE, Geidzinski E, Tran KK, Yu L, Zhou YH, Limoli CL. Irradiation of primary human gliomas triggers dynamic and aggressive survival responses involving microvesicle signaling. Environmental and molecular mutagenesis. 2016;57(5):405-15.

22. Sologuren I, Rodriguez-Gallego C, Lara PC. Immune effects of high dose radiation treatment: implications of ionizing radiation on the development of bystander and abscopal effects. Translational Cancer Research. 2014;3(1):18-31.

23. Vénéreau E, Ceriotti C, Bianchi ME. DAMPs from Cell Death to New Life. Front Immunol. 2015;6:422.

24. Escudier B, Dorval T, Chaput N, Andre F, Caby MP, Novault S, et al. Vaccination of metastatic melanoma patients with autologous dendritic cell (DC) derived-exosomes: results of the first phase I clinical trial. J Transl Med. 2005;3(1):10.

25. Morse MA, Garst J, Osada T, Khan S, Hobeika A, Clay TM, et al. A phase I study of dexosome immunotherapy in patients with advanced non-small cell lung cancer. J Transl Med. 2005;3(1):9.

26. Tomayko, M. M., & Reynolds, C. P. (1989). Determination of subcutaneous tumor size in athymic (nude) mice. Cancer Chemother Pharmacol, 24(3), 148-154.

27. Brondani Da Rocha A, Regner A, Grivicich I, Pretto Schunemann D, Diel C, Kovaleski G, Brunetto De Farias C, Mondadori E, Almeida L, Braga Filho A, Schwartsmann G. Radioresistance is associated to increased Hsp70 content in human glioblastoma cell lines. Int J Oncol. 2004 Sep;25(3):777-85.

28. Paolini A, Pasi F, Facoetti A, Mazzini G, Corbella F, Di Liberto R, Nano R. Cell death forms and HSP70 expression in U87 cells after ionizing radiation and/or chemotherapy. Anticancer Res. 2011 Nov;31(11):3727-31.

29. Francesca Pasi, Alessandro Paolini, Rosanna Nano, Riccardo Di Liberto, and Enrica Capelli. Effects of Single or Combined Treatments with Radiation and Chemotherapy on Survival and Danger Signals Expression in Glioblastoma Cell Lines. BioMed Research International, Volume 2014, Article ID 453497, 9 pages.

30. Rubner Y, Muth C, Strnad A, Derer A, Sieber R, Buslei R, Frey B, Fietkau R, Gaipl U. Fractionated radiotherapy is the main stimulus for the induction of cell death and of Hsp70 release of p53 mutated glioblastoma cell lines. Radiat Oncol. 2014 Mar 30;9(1):89.

31. Shevtsov MA, Nikolaev BP, Ryzhov VA, Yakovleva LY, Marchenko YY, Parr MA, Rolich VI, Mikhrina AL, Dobrodumov AV, Pitkin E, Multhoff G. Ionizing radiation improves glioma-specific targeting of superparamagnetic iron oxide nanoparticles conjugated with cmHsp70.1 monoclonal antibodies (SPION-cmHsp70.1). Nanoscale. 2015 Dec 28;7(48):20652-64.

## Claims

1. Microvesicles derived from in vitro irradiated neoplastic cells, **characterized by** having a mean size between 200 and 400 nm.

2. The microvesicles according to claim 1, wherein the mean size is of about 340 nm.

3. The microvesicles according to claims 1 and 2, wherein the radiation dose applied to obtain the microvesicles is between 45 and 55 Gy.

**4.** The microvesicles according to any of claims 1 to 3, wherein the radiation dose applied to obtain the microvesicles is 50 Gy.

**5.** The microvesicles according to any of claims 1 to 4, further **characterized in that** they do not contain Nuclear Factor of Activated T Cell 4 (NFATC4).

**6.** The microvesicles according to any of claims 1 to 5, further **characterized in that** they comprise HSP70 and/or HSP90 heat shock proteins.

**7.** The microvesicles according to any of claims 1 to 6, further **characterized in that** the outer membrane of the microvesicles contains phosphatidylserine.

**8.** The microvesicles according to any of claims 1 to 7, further **characterized in that** they are positive to annexin V.

**9.** The microvesicles according to any of the preceding claims, for use alone or in combination with one or more antineoplastic treatments in the treatment or prophylaxis of malignant neoplasms, or in the modulation of the antitumor immune response.

**10.** The microvesicles for use according to claim 9, wherein the one or more antineoplastic treatments are selected from the group consisting of: chemotherapy, radiotherapy, immunotherapy, or combinations thereof.

**12.** The microvesicles according to any of claims 1 to 8, for use as prognostic or diagnostic markers of neoplasms.

**13.** A therapeutic vaccine for cancer, **characterized in that** it comprises the microvesicles according to any of claims 1 to 8, along with pharmaceutically acceptable additives.

**14.** A process to prepare the microvesicles according to claim 1 or to prepare the therapeutic vaccine according to claim 13, wherein said process comprises the step of irradiating neoplastic cells at a radiation dose of between 45 and 55 Gy .

**15.** The process according to the preceding claim wherein the radiation dose is 50 Gy.

Figure 1

Figure 2

Figure 3

Figure 4

Figure 5

Figure 6

Figure 7

Figure 8

Figure 9

Figure 10

## INTERNATIONAL SEARCH REPORT

| International application No. |
| --- |
| PCT/IB2018/055740 |

**A. CLASSIFICATION OF SUBJECT MATTER**

**See extra sheet**

According to International Patent Classification (IPC) or to both national classification and IPC

**B. FIELDS SEARCHED**

Minimum documentation searched (classification system followed by classification symbols)
A61K, A61P, C12N

Documentation searched other than minimum documentation to the extent that such documents are included in the fields searched

Electronic data base consulted during the international search (name of data base and, where practicable, search terms used)

EPODOC, WPI, MEDLINE, EMBASE, BIOSIS

**C. DOCUMENTS CONSIDERED TO BE RELEVANT**

| Category* | Citation of document, with indication, where appropriate, of the relevant passages | Relevant to claim No. |
| --- | --- | --- |
| X | US 6703016 B1 (GREGOIRE MARC ET AL.) 09/03/2004, the whole document. In particular: page 4, column 7, lines 14 - 19; page 1 column 2, lines 48 - 49; page 2 column 4, lines 44 - 53; figures 7 - 8. | 1-15 |
| A | US 2015086639 A1 (HUANG BO) 26/03/2015, the whole document. | 1-15 |
| A | WO 2018085275 A1 (UNIV CALIFORNIA) 11/05/2018, the whole document. | 1-15 |
| A | WO 2017145162 A1 (BRODIE CHAYA ET AL.) 31/08/2017, the whole document. | 1-15 |
| A | US 2016310531 A1 (HUANG BO ET AL.) 27/10/2016, the whole document. | 1-15 |

☒ Further documents are listed in the continuation of Box C.    ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
| --- | --- |
| 13/12/2018 | **(17/12/2018)** |
| Name and mailing address of the ISA/ <br><br> OFICINA ESPAÑOLA DE PATENTES Y MARCAS <br> Paseo de la Castellana, 75 - 28071 Madrid (España) <br> Facsimile No.: 91 349 53 04 | Authorized officer <br> M. Hernández Cuéllar <br><br><br> Telephone No. 91 3498409 |

Form PCT/ISA/210 (second sheet) (January 2015)

## INTERNATIONAL SEARCH REPORT

| International application No. |
|---|
| PCT/IB2018/055740 |

| C (continuation). | DOCUMENTS CONSIDERED TO BE RELEVANT |
|---|---|

| Category * | Citation of documents, with indication, where appropriate, of the relevant passages | Relevant to claim No. |
|---|---|---|
| A | GIUSTI ILARIA ET AL. "Microvesicles as potential ovarian cancer biomarkers." BioMed research international (2013), Vol. 2013, Art. 703048, ISSN 2314-6141 (Electronic), <DOI: doi:10.1155/2013/703048 pubmed:23484144> | 1-15 |
| A | LENER THOMAS ET AL. "Applying extracellular vesicles based therapeutics in clinical trials - an ISEV position paper." Journal of extracellular vesicles (2015), Vol. 4, Pages 1-31, <DOI: doi:10.3402/jev.v4.30087 pubmed:26725829> | 1-15 |

Form PCT/ISA/210 (continuation of second sheet) (January 2015)

## INTERNATIONAL SEARCH REPORT

Information on patent family members

| International application No. |
| --- |
| PCT/IB2018/055740 |

| Patent document cited in the search report | Publication date | Patent family member(s) | Publication date |
| --- | --- | --- | --- |
| US2015086639 A1 | 26.03.2015 | US9844508 B2 | 19.12.2017 |
| | | CN103446580 A | 18.12.2013 |
| | | CN103446580B B | 03.08.2016 |
| | | WO2013178079 A1 | 05.12.2013 |
| WO2018085275 A1 | 11.05.2018 | NONE | |
| WO2017145162 A1 | 31.08.2017 | NONE | |
| US2016310531 A1 | 27.10.2016 | US2018071339 A1 | 15.03.2018 |
| | | CN106139148 A | 23.11.2016 |
| US6703016 B1 | 09.03.2004 | JP2002514408 A | 21.05.2002 |
| | | EP1078043 A1 | 28.02.2001 |
| | | WO9958645 A1 | 18.11.1999 |
| | | CA2331115 A1 | 18.11.1999 |
| | | AU4139899 A | 29.11.1999 |
| | | AU757443B B2 | 20.02.2003 |

Form PCT/ISA/210 (patent family annex) (January 2015)

22

## INTERNATIONAL SEARCH REPORT

International application No.

PCT/IB2018/055740

## CLASSIFICATION OF SUBJECT MATTER

*A61K39/00* (2006.01)
*A61P35/00* (2006.01)
*A61K41/00* (2006.01)
*C12N5/09* (2010.01)
*A61K38/00* (2006.01)

Form PCT/ISA/210 (extra sheet) (January 2015)

23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **OSTROM QT ; GITTLEMAN H ; FULOP J ; LIU M ; BLANDA R ; KROMER C et al.** CBTRUS Statistical Report: Primary Brain and Central Nervous System Tumors Diagnosed in the United States in 2008-2012. *Neuro-oncology,* 2015, vol. 17 (4), iv1-iv62
- **DEORAH S ; LYNCH CF ; SIBENALLER ZA ; RYKEN TC.** Trends in brain cancer incidence and survival in the United States: Surveillance, Epidemiology, and End Results Program, 1973 to 2001. *Neurosurgical focus,* 2006, vol. 20 (4), E1
- **LOUIS DN ; PERRY A ; REIFENBERGER G ; VON DEIMLING A ; FIGARELLA-BRANGER D ; CAVENEE WK et al.** The 2016 World Health Organization Classification of Tumors of the Central Nervous System: a summary. *Acta Neuropathol.,* 2016, vol. 131 (6), 803-20
- **OSTROM QT ; GITTLEMAN H ; FULOP J ; LIU M ; BLANDA R ; KROMER C et al.** CBTRUS Statistical Report: Primary Brain and Central Nervous System Tumors Diagnosed in the United States in 2008-2012. *Neuro-Oncology,* 2015, vol. 17 (4), iv1-iv62
- **LOPEZ-GONZALEZ MA ; SOTELO J.** Brain tumors in Mexico: characteristics and prognosis of glioblastoma. *Surgical neurology,* 2000, vol. 53 (2), 157-62
- **VELÁSQUEZ-PÉREZ L ; JIMÉNEZ-MARCIAL ME.** Clinical-histopathologic concordance of tumors of the nervous system at the Manuel Velasco Suarez National Institute of Neurology and Neurosurgery in Mexico City. *Arch Pathol Lab Med.,* 2003, vol. 127 (2), 187-92
- **STUPP R ; MASON WP ; VAN DEN BENT MJ ; WELLER M ; FISHER B ; TAPHOORN MJB et al.** Radiotherapy plus Concomitant and Adjuvant Temozolomide for Glioblastoma. *New England Journal of Medicine,* 2005, vol. 352 (10), 987-96
- **GRANER MW ; ALZATE O ; DECHKOVSKAIA AM ; KEENE JD ; SAMPSON JH ; MITCHELL DA et al.** Proteomic and immunologic analyses of brain tumor exosomes. *FASEB journal : official publication of the Federation of American Societies for Experimental Biology,* 2009, vol. 23 (5), 1541-57
- **VADER P ; BREAKEFIELD XO ; WOOD MJ.** Extracellular vesicles: emerging targets for cancer therapy. *Trends Mol Med.,* 2014, vol. 20 (7), 385-93

- **LEE Y ; EL ANDALOUSSI S ; WOOD MJ.** Exosomes and microvesicles: extracellular vesicles for genetic information transfer and gene therapy. *Hum Mol Genet.,* 2012, vol. 21 (R1), R125-34
- **CHARGAFF E ; WEST R.** The biological significance of the thromboplastic protein of blood. *J Biol Chem.,* 1946, vol. 166 (1), 189-97
- **WOLF P.** The nature and significance of platelet products in human plasma. *Br J Haematol.,* 1967, vol. 13 (3), 269-88
- **FRIEND C ; MAROVITZ W ; HENIE G ; HENIE W ; TSUEI D ; HIRSCHHORN K et al.** Observations on cell lines derived from a patient with Hodgkin's disease. *Cancer Res.,* 1978, vol. 38 (8), 2581-91
- **COCUCCI E ; RACCHETTI G ; MELDOLESI J.** Shedding microvesicles: artefacts no more. *Trends Cell Biol.,* 2009, vol. 19 (2), 43-51
- **MURALIDHARAN-CHARI V ; CLANCY J ; PLOU C ; ROMAO M ; CHAVRIER P ; RAPOSO G et al.** ARF6-regulated shedding of tumor cell-derived plasma membrane microvesicles. *Curr Biol.,* 2009, vol. 19 (22), 1875-85
- **ZWAAL RF ; SCHROIT AJ.** Pathophysiologic implications of membrane phospholipid asymmetry in blood cells. *Blood,* 1997, vol. 89 (4), 1121-32
- **LIMA LG ; CHAMMAS R ; MONTEIRO RQ ; MOREIRA ME ; BARCINSKI MA.** Tumor-derived microvesicles modulate the establishment of metastatic melanoma in a phosphatidylserine-dependent manner. *Cancer Lett.,* 2009, vol. 283 (2), 168-75
- **LEE TH ; D'ASTI E ; MAGNUS N ; AL-NEDAWI K ; MEEHAN B ; RAK J.** Microvesicles as mediators of intercellular communication in cancer--the emerging science of cellular 'debris. *Semin Immunopathol,* 2011, vol. 33 (5), 455-67
- **ARSCOTT WT ; TANDLE AT ; ZHAO S ; SHABASON JE ; GORDON IK ; SCHLAFF CD et al.** Ionizing radiation and glioblastoma exosomes: implications in tumor biology and cell migration. *Translational oncology,* 2013, vol. 6 (6), 638-48
- **RATAJCZAK J ; MIEKUS K ; KUCIA M ; ZHANG J ; RECA R ; DVORAK P et al.** Embryonic stem cell-derived microvesicles reprogram hematopoietic progenitors: evidence for horizontal transfer of mRNA and protein delivery. *Leukemia,* 2006, vol. 20 (5), 847-56

- **BAULCH JE ; GEIDZINSKI E ; TRAN KK ; YU L ; ZHOU YH ; LIMOLI CL.** Irradiation of primary human gliomas triggers dynamic and aggressive survival responses involving microvesicle signaling. *Environmental and molecular mutagenesis,* 2016, vol. 57 (5), 405-15
- **SOLOGUREN I ; RODRIGUEZ-GALLEGO C ; LARA PC.** Immune effects of high dose radiation treatment: implications of ionizing radiation on the development of bystander and abscopal effects. *Translational Cancer Research,* 2014, vol. 3 (1), 18-31
- **VÉNÉREAU E ; CERIOTTI C ; BIANCHI ME.** DAMPs from Cell Death to New Life. *Front Immunol.,* 2015, vol. 6, 422
- **ESCUDIER B ; DORVAL T ; CHAPUT N ; ANDRE F ; CABY MP ; NOVAULT S et al.** Vaccination of metastatic melanoma patients with autologous dendritic cell (DC) derived-exosomes: results of the first phase I clinical trial. *J Transl Med.,* 2005, vol. 3 (1), 10
- **MORSE MA ; GARST J ; OSADA T ; KHAN S ; HOBEIKA A ; CLAY TM et al.** A phase I study of dexosome immunotherapy in patients with advanced non-small cell lung cancer. *J Transl Med.,* 2005, vol. 3 (1), 9
- **TOMAYKO, M. M. ; REYNOLDS, C. P.** Determination of subcutaneous tumor size in athymic (nude) mice. *Cancer Chemother Pharmacol,* 1989, vol. 24 (3), 148-154
- **BRONDANI DA ROCHA A ; REGNERA ; GRIV-ICICH I ; PRETTO SCHUNEMANN D ; DIEL C ; KO-VALESKI G ; BRUNETTO DE FARIAS C ; MONDA-DORI E ; ALMEIDA L ; BRAGA FILHO A.** Radioresistance is associated to increased Hsp70 content in human glioblastoma cell lines. *Int J Oncol.,* September 2004, vol. 25 (3), 777-85
- **PAOLINI A ; PASI F ; FACOETTI A ; MAZZINI G ; CORBELLA F ; DI LIBERTO R ; NANO R.** Cell death forms and HSP70 expression in U87 cells after ionizing radiation and/or chemotherapy. *Anticancer Res.,* November 2011, vol. 31 (11), 3727-31
- **FRANCESCA PASI ; ALESSANDRO PAOLINI ; ROSANNA NANO ; RICCARDO DI LIBERTO ; EN-RICA CAPELLI.** Effects of Single or Combined Treatments with Radiation and Chemotherapy on Survival and Danger Signals Expression in Glioblastoma Cell Lines. *BioMed Research International,* vol. 2014, 9
- **RUBNER Y ; MUTH C ; STRNAD A ; DERER A ; SIE-BER R ; BUSLEI R ; FREY B ; FIETKAU R ; GAIPL U.** Fractionated radiotherapy is the main stimulus for the induction of cell death and of Hsp70 release of p53 mutated glioblastoma cell lines. *Radiat Oncol.,* 30 March 2014, vol. 9 (1), 89
- **SHEVTSOV MA ; NIKOLAEV BP ; RYZHOV VA ; YAKOVLEVA LY ; MARCHENKO YY ; PARR MA ; ROLICH VI ; MIKHRINA AL ; DOBRODUMOV AV ; PITKIN E.** Ionizing radiation improves glioma-specific targeting of superparamagnetic iron oxide nanoparticles conjugated with cmHsp70.1 monoclonal antibodies (SPION-cmHsp70.1). *Nanoscale,* 28 December 2015, vol. 7 (48), 20652-64